# EUROPEAN PATENT APPLICATION

(11) **EP 2 392 299 A1**
(43) Date of publication of application: **07.12.2011**
(21) Application number: 11168967.5
(22) Date of filing: 07.06.2011
(51) Int. Cl.: A61F 11/08

(54) **Hearing protection device**

(30) Priority: 07.06.2010 NL 2004836
(71) Applicant: Right Angle B.V., 1791 LK Den Burg (NL)
(72) Inventor: De Waal, Hubertus Adrianus, 1792 CP Oudeschild (NL)
(74) Representative: Van Breda, Jacobus

(57) **Abstract**

Ear silencer comprising an ear protector (2) hich in use extends in a part of a person's auditory duct, a faceplate (4) connected to said ear protector, which faceplate in use abuts against said person's external ear and which faceplate is provided with a membrane (6) that has sound damping properties.

## Description

When working under noisy conditions, or in general being present for a longer time in a noisy environment may damage a person's hearing capabilities. Normally such a person who is endangered to lose or suffer from an impaired hearing capacity may wear sound damping earphones or apply other means to restrain that high sound level pressures will travel to the eardrum at the end of such person's auditory duct.

A problem of the known means to damp said high sound level pressures is that they are indiscriminate as to the damping of high, mid and low frequencies. This deteriorates communication with other people and is also detrimental to respond appropriately to auditory information that is addressed to the person that is wearing these damping means.

It is an object of the invention to alleviate this problem and to provide an alternative for existing damping means that are used for restricting sound level pressures.

EP-A-0 315 942 discloses an ear silencer comprising a faceplate connected to an ear protector, which is provided with a hollow cylinder at the side facing away of the ear protector, which faceplate in use abuts against a person's external ear and which faceplate is provided with a membrane that has sound damping properties.

According to the invention an ear silencer is proposed in accordance with one or more of the appended claims.

In a first aspect of the invention the hollow cylinder cooperates with the faceplate for keeping the membrane in place and under tension by arranging that the hollow cylinder is connectable to the faceplate by a loosenable click-connection, which click-connection between the hollow cylinder and the faceplate operates for placing the membrane under tension.

The ear silencer of the invention may comprise an ear protector which in use extends in a part of a person's auditory duct. This is however merely an option and not a requirement.

The ear silencer of the invention can effectively be implemented with a balanced frequency damping characteristic, having emphasis at the higher frequencies. This secures that communication with the environment is hardly impaired, so that even entertaining a discussion with another person remains possible under noisy conditions.

The hollow cylinder may at least in part be filled with damping material, and the material to be used for the membrane can be of any suitable type, particularly material that is permeable for air.

The invention will hereinafter be further elucidated with reference to the drawing of an exemplary embodiment of the ear silencer of the invention.

In the drawing:
- figure 1 shows an exploded view of the ear silencer of the invention according to a first embodiment;
- figure 2 shows a part of the assembled ear silencer of the first embodiment;
- figure 3 shows the ear silencer of the first embodiment of the invention in a side view;
- figure 4 and 5 show the ear silencer of the first embodiment of the invention in use;
- figure 6 and 7 show a cross-section of the ear silencer according to the first embodiment of the invention;
- figures 8, 9, and 10 show in sequence some steps to assemble the ear silencer according to the first embodiment of the invention;
- figure 11 shows an exploded view of the ear silencer of the invention according to a second embodiment;
- figure 12 shows a part of the assembled ear silencer of the second embodiment;
- figure 13 shows the ear silencer of the second embodiment of the invention in a side view;
- figure 14 and 15 show the ear silencer of the second embodiment of the invention in use;
- figure 16 and 17 show a cross-section of the ear silencer according to the second embodiment of the invention;
- figures 18, 19a, and 20a show in sequence some steps to assemble the ear silencer according to the second embodiment of the invention; and
- figures 19b, and 20b show details of figures 19a and 20a.

Wherever in the figures the same reference numerals are applied, these numerals refer to the same parts.

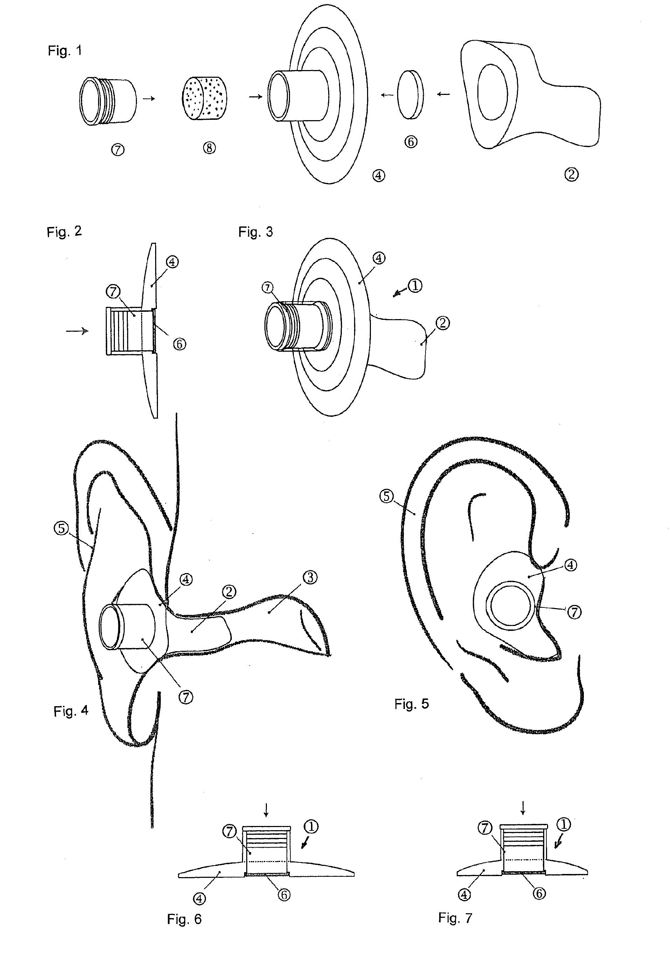

Referring first to figure 3 the ear silencer 1 according to a first embodiment of the invention is shown in a perspective view. With reference also to figure 1 pertaining to an exploded view of the ear silencer according to this first embodiment, the ear silencer is shown to comprise an ear protector 2 which is however optional and can be dispensed with. When it is used however, the ear protector 2 extends in a part of a person's auditory duct 3, as is clearly shown in figure 4.

Figure 1 and figure 3 show further that the ear silencer 1 of the first embodiment comprises a faceplate 4, which faceplate 4 in use abuts against said person's external ear 5 as shown in figure 4 and figure 5.

The faceplate 4 is provided with a membrane 6 that has sound damping properties. The location of the membrane 6 in the ear silencer of the first embodiment is clearly shown in figure 2. Figure 2 like figures 1 and 3 also shows that part of the ear silencer 1 is a hollow cylinder 7 at the side facing away of the ear protector 2. This hollow cylinder 7 may be filled with damping material 8 as is shown in figure 1.

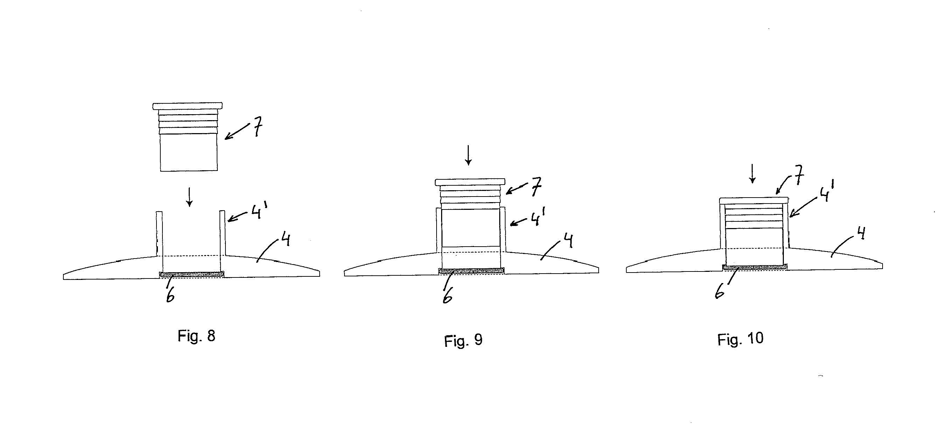

According to this first embodiment of the ear silencer 1 of the invention the hollow cylinder 7 cooperates with the faceplate 4 for keeping the membrane 6 in place. Preferably the hollow cylinder 7 cooperates with the faceplate 4 also for placing the membrane 6 under tension. Figures 8, 9, and 10 progressively show the placement of the hollow cylinder 7 such that it is connected to the faceplate 4. The faceplate 4 has for this purpose a receiving end 4' in which the hollow cylinder 7 can be clampingly received. When the hollow cylinder 7 has arrived at its eventual position as shown in figure 10 it clamps the membrane 6 and maintains it at the right tension to have it operate in accordance with the invention with the intended sound damping properties.

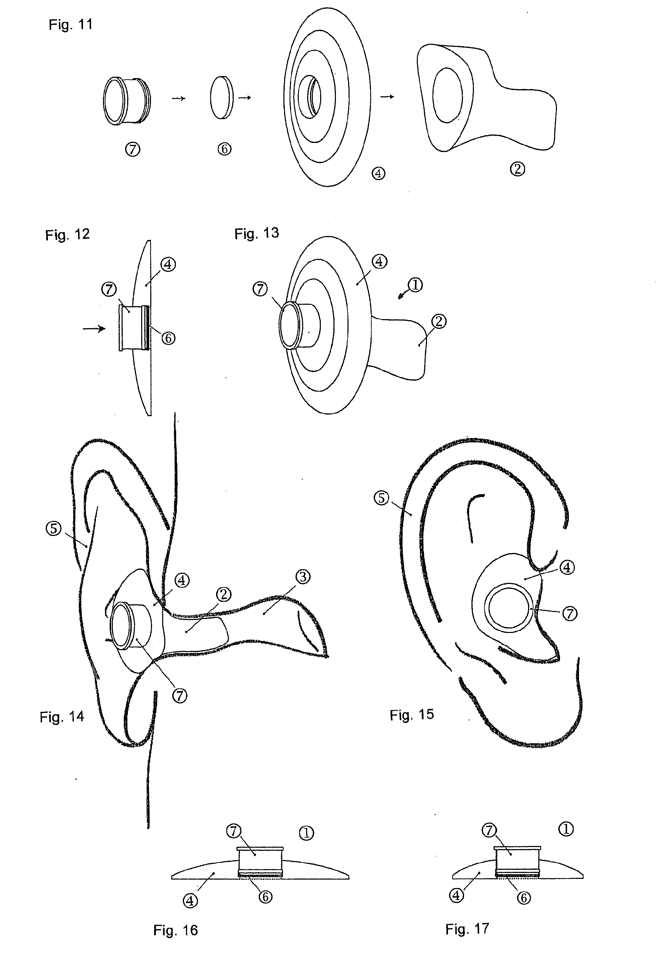

Referring now to figure 13 the ear silencer 1 according to a second embodiment of the invention is shown in a perspective view. With reference also to figure 11 pertaining to an exploded view of the ear silencer according to this second embodiment, the ear silencer is shown to comprise an ear protector 2 which is also optional in this embodiment, and can be dispensed with. When it is used however, like in the first embodiment the ear protector 2 extends in a part of a person's auditory duct 3, as is clearly shown in figure 14.

Figure 11 and figure 13 show further that the ear silencer 1 of the second embodiment comprises a faceplate 4, which faceplate 4 in use abuts against said person's external ear 5 as shown in figure 14 and figure 15.

The faceplate 4 is provided with a membrane 6 that has sound damping properties. The location of the membrane 6 in the ear silencer of the second embodiment is clearly shown in figure 12. Figure 12 like figures 11 and 13 also shows that part of the ear silencer 1 is a hollow cylinder 7 at the side facing away of the ear protector 2.

According to this second embodiment of the ear silencer 1 of the invention the hollow cylinder 7 cooperates with the faceplate 4 for keeping the membrane 6 in place, and preferably the hollow cylinder 7 cooperates with the faceplate 4 also for placing the membrane 6 under tension.

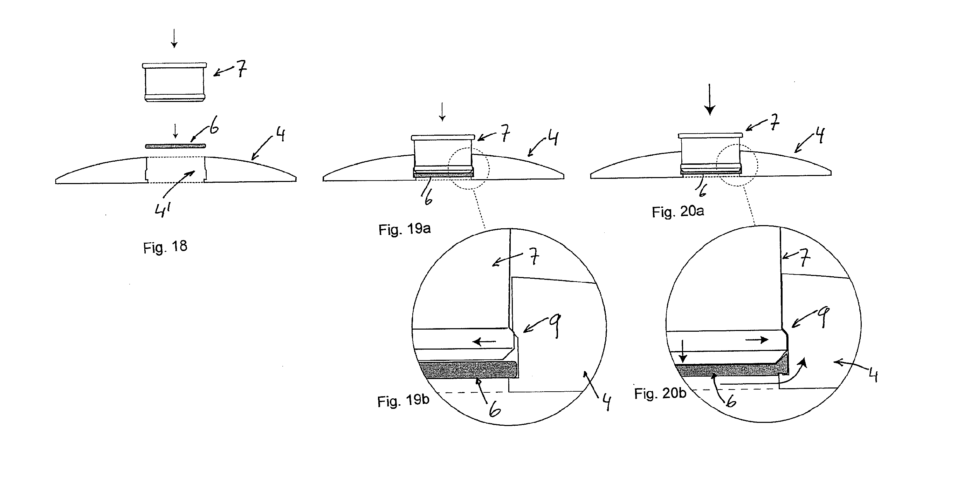

Figures 18, 19a, and 20a progressively show the placement of the hollow cylinder 7 such that it is connected to the faceplate 4. The faceplate 4 has for this purpose a receiving end 4' (see figure 18) in which the hollow cylinder 7 can be received.

In this second embodiment the receiving end 4' of the faceplate 4 is suitably arranged to cooperate with the hollow cylinder 7 such that the latter is connectable to the faceplate 4 by a loosenable click-connection 9. This is clearly shown in the detailed figures 19b and 20b. Both figures show the ear silencer of the invention with particular emphasis on the click-connection 9. The operation of the click-connection 9 secures that the membrane 6 is placed under tension once the hollow cylinder 7 has arrived at its final position in the receiving end 4' of the faceplate 4 as shown in figure 20b.

The sequential operation of maneuvering the hollow cylinder 7 from an external position with respect to the faceplate 4 to a position close to its eventual position as shown in figure 19b, and eventually to its final position as shown in figure 20b, arranges that the combined working of the hollow cylinder 7 with the faceplate 4 and the membrane 6 results in that the membrane 6 is fixed into its intended position when the click-connection 9 is complete. Figure 20b also shows that by this operation of the click-connection 9 said membrane 6 is placed under the required tension.

Still another way of embodying the ear silencer of the invention is to arrange that the membrane 6 is glued to the faceplate 4, in particular that the membrane 6 is glued to the faceplate 4 under pretension. This is not particularly shown in the figures, however, the manner in which this is to be implemented is clear for the person skilled in the art and a further discussion thereof can therefore be dispensed with.

## Claims

1. Ear silencer (1) comprising a faceplate (4) connected to an ear protector (2) and provided with a hollow cylinder (7) at the side facing away of the ear protector (2), which faceplate (4) in use abuts against said person's external ear (5) and which faceplate (4) is provided with a membrane (6) that has sound damping properties, **characterized in that** the hollow cylinder (7) cooperates with the faceplate (4) for keeping the membrane (6) in place and under tension, that the hollow cylinder (7) is connectable to the faceplate (4) by a loosenable click-connection (9), and that the click-connection (9) between the hollow cylinder (7) and the faceplate (4) operates for placing the membrane (6) under tension.

2. Ear silencer according to claim 1, **characterized in that** the hollow cylinder (7) is at least in part filled with damping material (8).

3. Ear silencer according to anyone of claims 1-7, **characterized in that** in use the ear protector (2) extends in a part of a person's auditory duct (3),
